(19) Europäisches Patentamt
European Patent Office
Office européen des brevets

(11) Veröffentlichungsnummer: **0 163 225**
**B2**

(12) **NEUE EUROPÄISCHE PATENTSCHRIFT**

(45) Veröffentlichungstag der neuen Patentschrift: **30.05.90**

(51) Int. Cl.$^5$: **C 07 C 303/00,** C 07 C 303/26, C 07 C 309/78

(21) Anmeldenummer: **85106136.6**

(22) Anmeldetag: **18.05.85**

(54) **Verfahren zur Herstellung von Acyloxybenzolsulfonsäuren und ihrer Alkali- und Erdalkalisalze.**

(30) Priorität: **26.05.84 DE 3419795**

(43) Veröffentlichungstag der Anmeldung: **04.12.85 Patentblatt 85/49**

(45) Bekanntmachung des Hinweises auf die Patenterteilung: **07.01.87 Patenblatt 87/02**

(45) Bekanntmachung des Hinweises auf die Entscheidung über den Einspruch: **30.05.90 Patentblatt 90/22**

(84) Benannte Vertragsstaaten: **AT BE CH DE FR GB IT LI NL SE**

(56) Entgegenhaltungen:
**EP-A-0 140 251**
**DE-A-1 618 012**
**DE-A-3 141 454**
**DE-C- 666 626**
**DE-C- 859 451**
**DE-C-3 337 921**
**GB-A-1 563 994**
**US-A-3 503 888**
**US-A-3 772 389**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Balzer, Wolf-Dieter, Dr.**
**Bruesseler Ring 34**
**D-6700 Ludwigshafen (DE)**
Erfinder: **Bechtolsheimer, Hans-Heinrich, Dr.**
**Ringstrasse 7**
**D-6521 Dittelsheim-Hessloch (DE)**
Erfinder: **Beyer, Karl-Heinz, Dr.**
**Knietschstrasse 6**
**D-6710 Frankenthal (DE)**
Erfinder: **Fikentscher, Rolf, Dr.**
**Von-Stephan-Strasse 27**
**D-6700 Ludwigshafen (DE)**
Erfinder: **Perner, Johannes, Dr.**
**Ginsterweg 4**
**D-6730 Neustadt (DE)**
Erfinder: **Widder, Rudi, Dr.**
**In der Taesch 7**
**D-6706 Leimen (DE)**
Erfinder: **Wolf, Helmut**
**Im Zollstock 6**
**D-6733 Hassloch (DE)**

**EP 0 163 225 B2**

Courier Press, Leamington Spa, England.

(56) Entgegenhaltungen:
CHEMICAL ABSTRACTS, Band 79, Nr. 1, 9. Juli 1973, Columbus, Ohio, USA; H. YAMAGUCHI "Adducts of tertiary amines with sulfur trioxide", Seite 424, Zusammenfassungsnr. 4991c

HOUBEN WEYL "Methoden der organischen Chemie" 4. Auflage, 1955,, GEORG THIEME VERLAG, Stuttgart Band IX, Seiten 503-508

INTERSCIENCE PUBLISHERS 1965, New York, USA; E.E. GILBERT "Sulfonation and Related Reactions", Seiten 7-20

Houben-Weyl, Methoden der organischen Chemie, 4. Auflage, Band IX (1955), S. 471-72

Römpp Chemie Lexikon, 6. Auflage (1966). S. 1994

E.E. Gilbert "Sulfonation and Related Reactions", S. 7-20 Interscience Publ., 1965, New York

L. Habicht "Die direkte Veresterung von Isethionsäure mit Fettsäuren im Hinblick auf Verbindungen des Igepon A-Typs" III. Int. Kongr. f. grenzfl. akt. Stoffe, Köln 1960, Untergruppe A/I/3, S. 116-118

Houben-Weyl, Methoden der Organischen Chemie, Bd. VIII (1952), S. 543-544

Ullmanns Encyclopädie der technischen Chemie, 4. Auflage., Bd. 22 (1982), S. 475, 481, 482

Chemical Reviews, Bd. 62(1962) S. 573-574

**Beschreibung**

Die Erfindung betrifft ein Verfahren zur Herstellung von Acyloxybenzolsulfonsäuren und ihrer Alkali- und Erdalkalisalze durch Sulfonierung eines Phenylesters ggf. in Gegenwart einer geringen Menge eines Komplexbildners für das sulfonierende Agens $SO_3$ oder Chlorsulfonsäure, anschliessende Nachacylierung und ggf. Neutralisation zum Alkali- oder Erdalkalisalz und ggf. vor dem Trocknen dessen oxydative Bleiche.

Es ist bekannt, dass Acyloxybenzolsulfonsäuren als aktivierte Ester Acylierungsmittel für Amine, Mercaptane, Wasserstoffperoxid und andere Verbindungen mit aktivem Wasserstoff sind. Für manche Anwendungen, wie die Acylierung von Feststoffen oder von wasserunlöslichen polymeren Verbindungen oder bei der Anwendung in Waschmitteln als Kaltbleichaktivatoren, beispielsweise gemäss EP-Anmeldung 28432, GB—PS 864 798, US—PS 4 412 934 oder der DE—AS 26 02 510, sind wasserlösliche Acylierungsmittel, wie die Salze von Acyloxybenzolsulfonsäuren, z.B. die bekannten Benzoyl- oder Acetyl-p-oxybenzolsulfonate, zweckmässig.

Weiterhin geht beispielsweise aus der US—PS 3 503 888 die Verwendung von Salzen von Acyloxybenzolsulfonsäuren in Toilettenseifen hervor. In der US—PS 3 503 888 wird eine Verfahrensweise zur Herstellung von Acyloxybenzolsulfonsäuren beschrieben, bei der man Phenol mit $SO_3$ sulfoniert und die erhaltene Phenolsulfonsäure mit einem Fettsäurechlorid verestert.

Wesentlich für die Herstellung von Acyloxybenzolsulfonsäuren ist, dass wegen ihrer Hydrolyseempfindlichkeit unter Ausschluss von Wasser gearbeitet werden muss. Aus diesem Grunde scheidet Schwefelsäure als übliches Sulfonierungsmittel aus. Auch die Sulfonierungen mit $SO_3$ oder Chlorsulfonsäure verlaufen in der Regel nicht optimal. Sie verlaufen üblicherweise unter Bildung von Gemischen aus o- und p-Isomeren und es entstehen eine Reihe unerwünschter Nebenprodukte, wie Sulfone und deren Folgeprodukte, durch Umacylierung entstehen Oxyketone und deren Folgeprodukte, ausserdem laufen Esterspaltungen ab. Die Nebenprodukte können eine spätere Aufarbeitung im Hinblick auf ein gut rieselfähiges Salz stören, da sie eine leichte Verbackbarkeit verursachen.

Der Monographie E. E. Gilbert, Sulfonation and Related Reactions, Interscience Publishers John Wiley a. Sons, New York, 1965, Kapitel 1, kann beispielsweise entnommen werden, dass $SO_3$ und Chlorsulfonsäure mit den verschiedensten organischen Verbindungen, wie Aminen, Pyridin, Ethern, Amiden u.a., Komplexe (meist 1:1-Addukte) bilden und dass diese Komplexe mildere Sulfonierungsreagentien sind als $SO_3$ oder Chlorsulfonsäure selbst. Durch diese Komplexbildung kann die Reaktivität des sulfonierenden Reagens beeinflusst werden. In der Regel laufen die Sulfonierungen mit diesen Komplexen bei höheren Temperaturen ab als ohne Komplexbildner und werden häufig in Gegenwart eines inerten Lösungsmittels oder eines Überschusses an Komplexbildner durchgeführt. Die Durchsicht der genannten Literaturstelle zeigt, dass die Reaktivität eines solchen Komplexes im Hinblick auf das zu sulfonierende Substrat nicht ohne weiteres vorausgesagt werden kann.

Aufgabe der Erfindung ist es, ein in technischem Massstab leicht durchzuführendes Verfahren zur Herstellung von Acyloxybenzolsulfonsäuren und ihrer Alkali- und Erdalkalisalze zur Verfügung zu stellen, durch das die gewünschten Sulfonsäuren in hoher Reinheit und in guten Ausbeuten zugänglich werden.

Gegenstand der Erfindung ist ein Verfahren zur Herstellung der Alkali- und Erdalkalisalze von Acyloxybenzolsulfonsäuren der Formel I

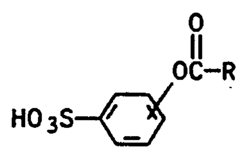 (I),

in der R einen geradkettigen oder verzweigten, gesättigten Alkylrest mit 5 bis 11 C-Atomen bedeutet, durch Sulfonierung eines Phenylesters, das dadurch gekennzeichnet ist, dass man einem Phenylester der Formel II

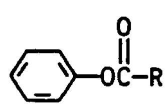 (II),

in der R die für Formel I angegebenen Bedeutungen hat in Gegenwart von 0,2 bis 30 Mol%, bevorzugt 1 bis 20 Mol%, eines Komplexbildners für $SO_3$ oder Chlorsulfonsäure, bezogen auf das $SO_3$ oder die Chlorsulfonsäure, mit $SO_3$ oder Chlorsulfonsäure bei Temperaturen von 20 bis 80°C, bevorzugt 25 bis 55°C, sulfoniert, das erhaltene Reaktionsgemisch nachacyliert mit einem Säurechlorid der Formel Cl—COR, in der R die für die Formel II genannten Bedeutungen hat, und anschliessend man zur Neutralisation die erhaltene flüssige Acyloxybenzolsulfonsäure der Formel I mit einer wäßrigen Lösung von Alkali- oder Erdalkali in Form des Hydoxids, Carbonats oder Bicarbonats in Wasser bei Temperaturen von 0 bis 60°C unter guter Durchmischung so zusammenbringt, daß ein pH-Wertbereich von 3,0 bis 5,5 eingehalten wird, und man ggf. das erhaltene Salz in an sich üblicher Weise aus der wäßrigen Lösung isoliert.

Die Herstellung der Ausgangsverbindungen der Formel II erfolgt zweckmässig durch direkte Umsetzung von Phenol mit einem Carbonsäurechlorid der Formel Cl—COR oder einem symmetrischen

Anhydrid der Formel RCO—O—COR, wobei R die für die Formel I angegebenen Bedeutungen hat, bei Temperaturen von 40 bis 60°C in an sich üblicher Weise.

Diese Veresterung lässt sich vorteilhaft auch in Form einer azeotropen Veresterung von Phenol mit einer Carbonsäure der Formel R—COOH, wobei R die für die Formel I angegebenen Bedeutungen hat, zweckmässigerweise in überschüssiger Menge von 5 bis 10 Mol.% und in Gegenwart eines aromatischen Kohlenwasserstoffs, wie Xylol oder Toluol, als Schleppmittel zum Auskreisen des entstehenden Wassers sowie eines stark sauren Veresterungskatalysators, wie p-Toluolsulfonsäure, Schwefelsäure, phosphoriger- oder unterphosphoriger Säure oder Mischungen dieser Säuren und anschliessender Destillation durchführen.

Die erfindungsgemässe Sulfonierung der Phenylester der Formel II erfolgt direkt mit $SO_3$ oder Chlorsulfonsäure ohne Lösungsmittel bei den oben angegebenen Temperaturen.

In einer besonders bevorzugten Ausführungsform erfolgt die Sulfonierung unter Zusatz von geringeren Mengen eines für das $SO_3$ oder die Chlorsulfonsäure komplexbildend wirkenden Agens, wie es beispielsweise der o.a. Monographie von Gilbert entnommen werden kann.

In Gegenwart dieser Zusätze führt die Sulfonierung bei relativ niederen Temperaturen zu Produkten hoher Reinheit. Erstaunlicherweise reagieren die bekannten 1:1-Addukte von $SO_3$ oder der Chlorsulfonsäure in dem erfindungsgemässen Temperaturbereich nicht oder nur sehr langsam. Zudem bereitet eine grössere Menge des eingesetzten Komplexbildners, z.B. eines Amins, zusätzlichen Aufwand bei der Aufarbeitung, wenn ein möglichst reines Endprodukt erhalten werden soll.

Im einzelnen seien als komplexbildend wirkende Verbindungen genannt Dioxan, Polyalkylenoxide, wie Diethylen- und Dipropylenglykol, deren Endgruppen durch Alkylreste mit 1 bis 18 C-Atomen verschlossen sind, Formamid, durch 1 oder 2 Alkylreste mit 1 bis 4 C-Atomen am Amidstickstoff substituierte aliphatische Carbonsäureamide mit 1 bis 10 C-Atomen, wie Dimethylformamid, Diethyl- oder Dibutylformamid, Benzoesäureamide, gegebenenfalls am Stickstoff durch einen Alkylrest mit 1 bis 4 C-Atomen substituierte cyclische 5- bis 7-gliedrige Amide, wie N-Methylpyrrolidon-2, N-Methylpiperidon-2, ε-Caprolactam, Triazinderivate, wie Melamin, Benzoguanamin, Acetoguanamin, Trialkylamine mit 1 bis 6 C-Atomen im Alkyl, N,N-$C_1$—$C_4$-Dialkyl-cyclohexylamine, Pyridin, Triphenylphosphin, Amidosulfonsäure, Imidazol oder Bortrifluorid. Gegebenenfalls können auch Mischungen dieser Komplexbildner verwendet werden.

Davon sind die N,N-disubstituierten Formamide mit 1 bis 4 C-Atomen im Alkyl, insbesondere das Dimethylformamid, und das 1,4-Dioxan besonders bevorzugt.

Zweckmässigerweise wird die komplexierend wirkende Verbindung bei dem erfindungsgemässen Verfahren dem flüssigen Phenylester zugegeben.

Für R kommen als Alkylreste mit 5 bis 11 C-Atomen beispielsweise Pentyl, Heptyl, 2-Ethylpentyl, Octyl, verzweigte Octylreste, Undecyl, in Betracht.

Von den Resten für R sind besonders bevorzugt n-Heptyl, n-Octyl und 3,5,5-Trimethylpentyl.

Bei der Herstellung können anstelle der reinen Säurechloride zweckmässig die technisch erhältlichen Gemische, die in der Regel mindestens 95% definiertes Säurechlorid Cl—COR enthalten, eingesetzt werden. Dabei wird das 3,5,5-Trimethylhexansäurechlorid im technischen Sprachgebrauch häufig als Isononansäurechlorid bezeichnet.

Im übrigen steht in der Formel I der Rest —O—COR aufgrund des entstehenden Reaktionsgemisches bevorzugt in der p-Stellung mit einem Anteil an o-Verbindung.

Bei der Umsetzung des zu sulfonierenden Phenylesters mit $SO_3$ oder Chlorsulfonsäure wird vorteilhaft ein Molverhältnis von etwa 1:1 eingehalten, um beispielsweise Sulfonbildung und Disulfonierungen und weitere Nebenprodukte zu vermeiden. Die Abweichungen von der genauen molaren Menge beträgt zweckmässig nicht mehr als 5 Mol% an Überschuss des Sulfonierungsmittels.

Der bei Verwendung von Chlorsulfonsäure entstehende Chlorwasserstoff kann ohne Schwierigkeiten durch Ausgasen, z.B. unter vermindertem Druck bei 10 bis 40 mbar, praktisch vollständig entfernt werden.

Die hier beschriebenen Sulfonierungen einschliesslich der Veresterungen können diskontinuierlich oder kontinuierlich durchgeführt werden.

Bei der kontinuierlichen Ausführungsform kann man beispielsweise so vorgehen, dass man die Reaktionskomponenten in einem Rohrreaktor oder einer Rührkesselkaskade zusammenführt.

In der erhaltenen rohen Acyloxybenzolsulfonsäure lassen sich in der Regel Produkte mit freien phenolischen Hydroxylgruppen nachweisen, die durch bei der Sulfonierung als Nebenreaktion ablaufende Esterspaltungen entstehen. Ihre Menge kann potentiometrisch bestimmt und durch eine äquimolare Menge des Carbonsäurechlorids der Formel Cl—COR, in der R die für Formel I angegebenen Bedeutungen hat, wieder verestert werden. Diese Umsetzung wird bei 40 bis 50°C in 2 bis 3 Stunden durchgeführt.

Aus praktischen Gründen und wegen der Zersetzlichkeit der gewonnenen Acyloxybenzolsulfonsäuren, die als aktivierte Phenolester sehr hydrolyse-empfindlich sind, ist es vorteilhaft, die erhaltenen flüssigen Acyloxybenzolsulfonsäuren in ihre Alkali- oder Erdalkalisalze zu überführen. Davon ist das Natriumsalz besonders bevorzugt. Bei einer zweckmässigen und besonders vorteilhaften Neutralisation geht man so vor, dass man die flüssige Acyloxybenzolsulfonsäure mit einer wässerigen Lösung von Alkali- oder Erdalkali in Form des Hydroxids, Carbonats oder Bicarbonats in Wasser bei Temperaturen von 0 bis 60°C, bevorzugt 10 bis 50°C, unter guter Durchmischung so zusammenbringt, dass ein pH-Wert-Bereich von 2,5 bis 7,0, bevorzugt von 3,0 bis 5,5, eingehalten wird, und man gegebenenfalls das erhaltene Salz in an sich

üblicher Weise aus der wässerigen Lösung in fester Form isoliert.

Dieses spezielle Neutralisationsverfahren, bei dem Acyloxybenzolsulfonsäuren ohne nennenswerte Verseifung neutralisiert werden können, ist übrigens Gegenstand der nicht vorveröffentlichten deutschen Patentanmeldung P 33 37 921.1. Im einzelnen geht man so vor, dass man in Wasser gleichzeitig die flüssige Acyloxybenzolsulfonsäure und eine 5 bis 50 gew.%ige wässerige Lösung von Alkali- oder Erdalkali in Form des Hydroxids, Carbonats oder Bicarbonats bei Temperaturen von 0 bis 60°C, bevorzugt 10 bis 50°C, unter Rühren so einlaufen lässt, dass ein pH-Wert-Bereich von 2,5 bis 7,0, bevorzugt 3,0 bis 5,5, eingehalten wird.

Diese Neutralisation kann diskontinuierlich oder kontinuierlich durchgeführt werden. Bei der kontinuierlichen Arbeitsweise werden die Komponenten Wasser, Acyloxybenzolsulfonsäure und Alkali in einem statischen oder dynamischen Mischer zusammengeführt.

Durch dieses Neutralisationsverfahren können stabile wässerige Lösungen der Acyloxybenzolsulfonate in Konzentrationen von 20 bis 60 Gew.% hergestellt werden. Aus diesen Lösungen können die reinen Salze in an sich üblicher Weise, beispielsweise durch Eindampfen, Walzentrocknen, Sprühtrocknen, Gefriertrocknen oder Wirbelbetttrocknen isoliert werden. In einer besonders bevorzugten Ausführungsform wird diese spezielle Neutralisation in Gegenwart von 1 bis 2 Gew.%, bezogen auf die Acyloxybenzolsulfonsäure, von einem wasserlöslichen Phosphat, Phosphit, Tartrat oder einem Komplexbildner für Schwermetalle oder einem Polymeren aus Acrylsäure und/oder Maleinsäure durchgeführt. Dabei werden in der Regel die wasserlöslichen Natriumsalze verwendet.

Die wässerigen Lösungen sind dann wesentlich weniger gefärbt und neigen bei der Weiterverarbeitung weniger zu Verfärbungen, wenn man die Neutralisation in Gegenwart dieser Stoffe durchführt. Im einzelnen seien beispielsweise genannt: Natriumdihydrogenphosphat, Dinatriumtartrat, Natriumhydrogentartrat, Natriumphosphit, unterphosphorige Säure, Nitrilotriessigsäure, Ethylendiamintetraessigsäure, Diethylentriaminpentaessigsäure, Hydroxyethylethylendiamintriessigsäure, Nitrilotrimethylenphosphonsäure oder Polycarbonsäuren aus Acrylsäure und/oder Maleinsäure und ihre Natriumsalze. Die verwendeten Polyacrylsäuren weisen K-Werte von 15 bis 120 und die Acrylsäure-Maleinsäure-Copolymerisate K-Werte von 30 bis 100 auf, jeweils gemessen als vollständig neutralisiertes Na-Salz in 1 gew.-%iger wässeriger Lösung bei 25°C.

Es wird darauf hingewiesen, dass bei der Sulfonierung von Phenylestern nach dem erfindungsgemässen Verfahren Nebenprodukte entstehen, die in der Folge insbesondere bei der Anwendung von Chlorsulfonsäure zu farbigen Verbindungen kondensieren und auch zu Verfärbungen des sprühgetrockneten Produktes Anlass geben. Diese Verunreinigungen lassen sich durch Zugabe von Natriumperborat, Wasserstoffperoxid oder Natriumperoxodisulfat zur neutralisierten Lösung in einer Menge von 0,05 bis 2 Gew.%, bezogen auf den Feststoffgehalt der wässerigen Lösung, und Erwärmen der Lösung auf 40 bis 80°C, bevorzugt 45 bis 55°C innerhalb von 5 bis 15, bevorzugt 8 bis 11 Stunden, oxydativ aufhellen. Anschliessend kann dann getrocknet werden.

## Beispiele

Die vorliegenden Beispiele werden mit dem 3,5,5-Trimethylpentylrest für R durchgeführt, soweit nichts anderes vermerkt ist. Andere Acylreste verhalten sich völlig analog. Der Gehalt der Reaktionsmischung an Acyloxybenzolsulfonsäure wird zweckmässigerweise nach der Neutralisation mit wässeriger Natronlauge und Sprühtrocknung an den isolierten Natriumsalzen durch Zweiphasentitration nach DIN/ISO 2271 bestimmt. Teile sind Gewichtsteile.

### Allgemeine Vorschriften

A. *Herstellung der Phenylester als Ausgangsverbindungen der Formel II*

1. 94 Teile Phenol werden geschmolzen und bei 45°C werden unter Rühren 176 Teile 3,5,5-Trimethylhexansäurechlorid (Isononansäurechlorid) innerhalb von 1 bis 2 Stunden zugegeben. Es wird 1 Stunde bei 50°C nachgerührt.

2. 158 Teile Isononansäure, 103 Teile Phenol und 60 Teile Toluol werden mit 1 Teil p-Toluolsulfonsäure und 0,3 Teilen unterphosphoriger Säure zum Sieden erhitzt. Durch azeotrope Destillation bei 140 bis 160°C werden 15 Teile Wasser ausgetragen. Danach werden das Toluol und das überschüssige Phenol bei 20 mbar bis zu einer Temperatur von 140°C abdestilliert. Der erhaltene rohe Phenylester wird durch Destillation unter vermindertem Druck bei 140 bis 160°C und ca. 20 mbar gereinigt. Die Ausbeute beträgt 187 Teile = 80%.

B. *Sulfonierung der Phenylester ohne Komplexbildner (Vergleichsbeispiele 1 und 2)*

Zu 234 Teilen Isononansäurephenylester werden gemäss Tabelle entweder 122 Teile Chlorsulfonsäure oder 84 Teile Schwefeltrioxid bei einer Temperatur von 25 bis 55°C im Laufe von 1 bis 2 Stunden zugegeben. Es wird 1 Stunde bei 50°C nachgerührt und dann unter vermindertem Druck bei 10 bis 20 mbar der Chlorwasserstoff entfernt.

C. *Sulfonierung der Phenylester mit Komplexbildnern*

Zu 234 Teilen Isononansäurephenylester wird Komplexbildner gemäss Tabelle 1 zugegeben. Unter Kühlung lässt man 122 Teile Chlorsulfonsäure oder 84 Teile Schwefeltrioxid innerhalb von 1 bis 2 Stunden

so zulaufen, dass eine Temperatur von 55°C nicht überschritten wird. Es wird 1 Stunde bei 50°C nachgerührt und dann unter vermindertem Druck bei 10 bis 20 mbar der Chlorwasserstoff entfernt.

D. *Nachacylierung*

Durch Titration mit 1-normaler wässeriger Natronlauge werden mit Hilfe eines Metrohm-Titroprocessors (Hersteller Fa. Metrohm) die in der rohen Acyloxybenzolsulfonsäure enthaltenen phenolischen OH-Gruppen ermittelt und anschliessend mit der äquimolaren Menge Isononansäurechlorid 2 Stunden bei 50°C verestert. Hierzu werden 4 bis 20 Teile Isononansäurechlorid im Hinblick auf die Arbeitsweise gemäss B oder C benötigt.

E. *Neutralisation und oxidative Bleiche*

100 Teile der erhaltenen rohen Acyloxybenzolsulfonsäure lässt man unter gutem Rühren zu 100 Teilen Wasser zulaufen. Gleichzeitig wird 50 gew.%ige wässerige Natronlauge so zugetropft, dass sich in der wässerigen Lösung ein pH-Wert von 3,0 bis 5,5 (Verfolgung mittels Glaselektrode) einstellt. Die Temperatur der Reaktionsmischung wird durch Kühlung unter 50°C gehalten. Nach dem Ende der Zugabe der Acyloxybenzolsulfonsäure wird die Lösung auf einen pH-Wert von 5,5 eingestellt. Zur oxidativen Bleiche werden 0,1 Teile Natriumperborat zugegeben und die Lösung anschliessend 6 Stunden auf 50°C erwärmt. Das Natriumsalz wird durch Sprühtrocknung aus der wässerigen Lösung isoliert.

F. *Herstellung von n-Pentanoyl- und Undecanoyloxibenzolsulfonsäure-Natriumsalz*

1. 188 Teile Phenol werden geschmolzen und bei 45°C mit 269 Teilen Capronsäurechlorid (n-Pentancarbonsäurechlorid) innerhalb von 1 bis 2 Stunden versetzt. Es wird eine Stunde bei 50°C nachgerührt und danach werden 8 Teile Dimethylformamid zugefügt.

Innerhalb von 1 bis 2 Stunden lässt man 244 Teile Chlorsulfonsäure so zulaufen, dass eine Temperatur von 55°C nicht überschritten wird.

Es wird eine Stunde bei 50°C nachgerührt und dann unter vermindertem Druck bei 10 bis 20 mbar der restliche Chlorwasserstoff entfernt.

Die erhaltene rohe Sulfonsäure wird wie unter Beispielen D und E beschrieben, aufgearbeitet. Gehalt: 81,1% Na-Salz.

2. 141 Teile Phenol, 328 Teile Laurinsäurechlorid (Undecancarbonsäurechlorid), 8 Teile Dimethylformamid und 183 Teile Chlorsulfonsäure werden wie unter Beispiel F1 beschrieben miteinander umgesetzt und aufgearbeitet. Gehalt: 85,3% Aktivsubstanz (Na-Salz).

Tabelle: Beispiele nach den Verfahrensschritten B bis E

| Beisp. | $ClSO_3H$ | $SO_3$ | Komplexbildner | Tle. | Mol%[1] | Gehalt an Na-Salz[2] der Acyloxybenzol-sulfonsäure (%) |
|---|---|---|---|---|---|---|
| 1 | × | – | – | – | – | 75,7 |
| 2 | – | × | – | – | – | 74,3 |
| 3 | × | – | Dimethylformamid | 2,34 | 3,0 | 85,0 |
| 4 | × | – | Dimethylformamid | 4,68 | 6,1 | 84,1 |
| 5 | × | – | Dimethylformamid | 9,36 | 12,3 | 87,6 |
| 6 | × | – | Dimethylformamid | 14,04 | 18,3 | 86,4 |
| 7 | × | – | Dioxan | 4,68 | 5,0 | 86,7 |
| 8 | × | – | Dioxan | 14,04 | 15,2 | 86,5 |
| 9 | × | – | Dioxan | 23,4 | 25,3 | 84,5 |
| 10 | × | – | Harnstoff | 4,68 | 7,4 | 84,3 |
| 11 | × | – | Tetramethylharnstoff | 4,68 | 3,8 | 86,6 |
| 12 | × | – | Tetramethylharnstoff | 9,36 | 7,6 | 83,8 |
| 13 | × | – | Imidazol | 4,68 | 6,6 | 83,1 |
| 14 | × | – | Diisobutylformamid | 4,68 | 4,5 | 82,6 |
| 15 | × | – | Melamin | 2,34 | 1,8 | 81,8 |
| 16 | × | – | Benzoguanamin | 4,68 | 2,4 | 81,2 |
| 17 | × | – | N-Methylpyrrolidon | 4,68 | 4,0 | 82,4 |
| 18 | × | – | N-Methylpyrrolidon | 9,36 | 7,9 | 84,7 |
| 19 | × | – | Dimethylcyclohexylamin | 9,36 | 7,0 | 83,0 |
| 20 | × | – | Succinimid | 9,36 | 9,0 | 85,9 |
| 21 | × | – | Phthalimid | 9,36 | 6,0 | 82,7 |
| 22 | × | – | Triphenylphosphin | 9,36 | 3,4 | 84,5 |
| 23 | × | – | Ethylenglykoldimethylether | 4,68 | 5,0 | |
| 24 | – | × | Dimethylformamid | 11,17 | 14,6 | 80,9 |
| 25 | – | × | Triethylamin | 9,36 | 8,8 | 80,7 |
| 26 | – | × | Phthalimid | 9,36 | 6,0 | 78,7 |
| 27 | – | × | Tetramethylharnstoff | 9,36 | 7,6 | 79,5 |
| 28 | – | × | Dimethylcyclohexylamin | 9,36 | 7,0 | 77,3 |
| 29 | – | × | N-Methylpyrrolidon | 9,36 | 7,9 | 82,0 |
| 30 | × | – | Dimethylformamid | 44 | 57 | 71,8 |
| 31 | × | – | Dimethylformamid | 73 | 100 | 79,2 |
| 32 | – | × | Dimethylformamid | 73 | 100 | – |
| 33 | × | – | Harnstoff | 60 | 100 | – |

[1] bezogen auf sulfonierendes Agens
[2] Zweiphasentitration nach DIN/ISO 2271

Aus den zahlenmässigen Angaben der Tabelle geht hervor, dass der Zusatz von Komplexbildnern, insbesondere bei der Verwendung von Chlorsulfonsäure, zu einer Ausbeutesteigerung gegenüber ohne diesen Zusatz um ca. 11% führt.

Die Beispiele 30 bis 33 sind Vergleichsbeispiele, wozu erläutert wird: Die Verwendung von Dimethylformamid in einer Menge von 57 Mol% führt zu einem wesentlich geringeren Gehalt an Aktivsubstanz (Beispiel 30).

Zu Beispiel 31: Die Umsetzung mit der äquimolaren Menge Dimethylformamid ergab einen Aktivgehalt von 79,2%, wobei die Temperatur auf 90°C erhöht werden musste, da bei 55°C keine Reaktion erfolgte. Die höheren Temperaturen bewirken eine starke Dunkelfärbung des Reaktionsproduktes trotz Bleiche und Zusätze.

Bei den Beispielen 32 und 33 entstanden inhomogene Mischungen, die im erfindungsgemässen Temperaturbereich bis 80°C nicht reagierten.

**Patentansprüche**

1. Verfahren zur Herstellung der Alkali- und Erdalkalisalze von Acyloxybenzolsulfonsäuren der Formel I

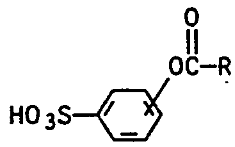

(I),

in der R einen geradkettigen oder verzweigten gesättigten Alkylrest mit 5 bis 11 C-Atomen bedeutet, durch Sulfonierung eines Phenylesters, dadurch gekennzeichnet, daß man einen Phenylester der Formel II

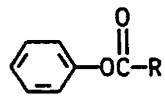

(II),

in der R die für Formel I angegebene Bedeutung hat in Gegenwart von 0,2 bis 30 Mol% eines Komplexbildners für $SO_3$ oder Chlorsulfonsäure, bezogen auf das $SO_3$ oder die Chlorsulfonsäure, mit $SO_3$ oder Chlorsulfonsäure bei Temperaturen von 20 bis 80°C sulfoniert, die erhaltene Acyloxybenzolsulfonsäure nachacyliert mit einem Säurechlorid der Formel Cl—COOR, in der R die für die Formel II genannten Bedeutungen hat und anschließend man zur Neutralisation die erhaltene flüssige Acyloxybenzolsulfonsäure der Formel I mit einer wäßrigen Lösung von Alkali- oder Erdalkali in Form des Hydoxids, Carbonats oder Bicarbonats in Wasser bei Temperaturen von 0 bis 60°C unter guter Durchmischung so zusammenbringt, daß ein pH-Wertbereich von 3,0 bis 5,5 eingehalten wird, und man ggf. das erhaltene Salz in an sich üblicher Weise aus der wäßrigen Lösung isoliert.

2. Verfahren nach Anspruch 1, dadurch gekennzeichnet, daß man einen Phenylester der Formel II direkt mit $SO_3$ oder Chlorsulfonsäure bei Temperaturen von 25 bis 55°C sulfoniert.

3. Verfahren nach Anspruch 1, dadurch gekennzeichnet, daß man die Sulfonierung eines Phenylesters der Formel II in Gegenwart von 1 bis 20 Mol% eines Komplexbildners für $SO_3$ oder Chlorsulfonsäure, bezogen auf das $SO_3$ oder die Chlorsulfonsäure, bei Temperaturen von 25 bis 55°C durchführt.

4. Verfahren nach Anspruch 1, dadurch gekennzeichnet, daß man das die Acyloxybenzolsulfonsäure enthaltende Reaktionsgemisch mit einer zu den vorhandenen freien phenolischen OH-Gruppen äquimolaren Menge an Carbonsäurechlorid der Formel Cl—COR, wobei R die für Formel I angegebenen Bedeutungen hat, bei 40 bis 50°C verestert.

5. Verfahren nach Anspruch 1, dadurch gekennzeichnet, daß die Neutralisation bei Temperaturen von 10 bis 40°C durchgeführt wird.

6. Verfahren nach Anspruch 1 und/oder 5, dadurch gekennzeichnet, daß die Neutralisation in Gegenwart von 1 bis 2 Gew.%, bezogen auf die Acyloxybenzolsulfonsäure, eines löslichen Phosphats, Phosphits, Tartrats, eines Komplexbildners für Schwermetallsalze oder eines Polymers aus Acrylsäure und/ oder Maleinsäure durchgeführt wird.

7. Verfahren nach den Ansprüchen 1, 5 oder 6, dadurch gekennzeichnet, daß man die neutralisierte Lösung mit 0,05 bis 2 Gew%, bezogen auf den Feststoffgehalt, Natriumperborat, Wasserstoffperoxid oder Natriumperoxidisulfat bei Temperaturen von 40 bis 80°C, innerhalb von 5 bis 15 Stunden oxidativ aufhellt.

8. Verfahren nach Anspruch 1, dadurch gekennzeichnet, daß man zur Sulfonierung einen Phenylester der Formel II einsetzt, der durch azeotrope Veresterung von Phenol mit einer Carbonsäure der Formel R—COOH, in der R die für die Formel I angegebenen Bedeutungen hat, in Gegenwart eines aromatischen Kohlenwasserstoffs sowie eines stark sauren Veresterungskatalysators hergestellt worden ist.

**Revendications**

1. Procédé de préparation des sels de métaux alcalins ou alcalino-terreux d'acides acyloxy-benzène-sulfoniques de la formule I

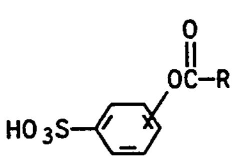

(I),

dans laquelle R désigne un radical alkyle saturé à chaîne droite ou ramifiée en $C_5$ à $C_{11}$, par sulfonation d'un ester phénylique, caractérisé en ce que l'on soumet un ester phénylique de la formule II

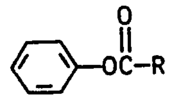 (II),

dans laquelle R possède l'une des significations définies pour la formule I, entre 20 et 80°C à une sulfonation par le $SO_3$ ou l'acide chlorosulfonique, en présence de 0,2 à 30% molaires, par rapport au $SO_3$ ou à l'acide chlorosulfonique, d'un agent complexant pour le $SO_3$ ou l'acide chlorosulfonique, puis l'acide acyloxy-benzène-sulfonique formé à une post-acylation par un chlorure d'acide de la formule Cl—COR, dans laquelle R possède l'une des significations définies pour la formule II, et ensuite pour neutraliser l'acide acyloxy-benzène-sulfonique liquide de la formule I obtenu, on y ajoute entre 0 et 60°C sous une bonne agitation une solution aqueuse d'un métal alcalin ou alcalino-terreux sous forme de son hydroxyde, de son carbonate ou de son hydrogénocarbonate dans de l'eau, en une proportion telle que le pH s'établit dans la gamme de 3,0 à 5,5, le sel formé pouvant éventuellement être isolé de manière usuelle de la solution aqueuse.

2. Procédé suivant la revendication 1, caractérisé en ce que l'on soumet un ester phénylique de la formule II à une sulfonation directe par le $SO_3$ ou l'acide chlorosulfonique entre 25 et 55°C.

3. Procédé suivant la revendication 1, caractérisé en ce que la sulfonation d'un ester phénylique de la formule II est réalisée entre 25 et 55°C en présence de 1 à 20% molaires, par rapport au $SO_3$ ou à l'acide chlorosulfonique, d'un agent complexant pour le $SO_3$ ou l'acide chlorosulfonique.

4. Procédé suivant la revendication 1, caractérisé en ce que l'on soumet le mélange réactionnel contenant l'acide acyloxy-benzène-sulfonique à une estérification entre 40 et 50°C à l'aide du chlorure d'un acide carboxylique de la formule Cl—COR, dans laquelle R possède l'une des significations définies pour la formule I, en une proportion équimolaire par rapport aux groupes hydroxyle phénoliques libres présents.

5. Procédé suivant la revendication 1, caractérisé en ce que la neutralisation est réalisée entre 10 et 40°C et à un pH compris entre 3,0 et 5,5.

6. Procédé suivant la revendication 1 et(ou) la revendication 5, caractérisé en ce que la neutralisation est réalisée en présence de 1 à 2% en poids, par rapport à l'acide acyloxy-benzène-sulfonique, d'un phosphate, phosphite ou tartrate soluble, d'un agent complexant les sels de métaux lourds ou d'un polymère de l'acide acrylique et(ou) de l'acide maléique.

7. Procédé suivant les revendications 1, 5 ou 6, caractérisé en ce que la solution neutralisée est soumise pendant cinq à quinze heures, entre 40 et 80°C, à un blanchiment oxydant à l'aide de 0,05 à 2% en poids, par rapport à la teneur en matière solide, de perborate de sodium, de peroxyde d'hydrogène ou de peroxodisulfate de sodium.

8. Procédé suivant la revendication 1, caractérisé en ce que l'on soumet à la sulfonation un ester phénylique de la formule II, préparé par une estérification azéotrope du phénol, en présence d'un hydrocarbure aromatique et d'un catalyseur d'estérification fortement acide, par un acide carboxylique de la formule R—COOH, dans laquelle R possède l'une des significations définies pour la formule I.

## Claims

1. A process for the preparation of the alkali metal and alkaline earth metal salts of an acyloxybenzenesulfonic acid of the formula I

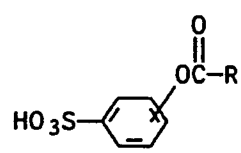 (I),

where R is straight-chain or branched saturated alkyl of 5 to 11 carbon atoms, by sulfonation of a phenyl ester, wherein a phenyl ester of the formula II

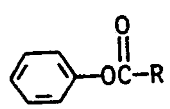 (II),

where R has the meanings given for formula I, in the presence of from 0.2 to 30 mole%, based on $SO_3$ or chlorosulfonic acid, of a complexing agent for $SO_3$ or chlorosulfonic acid, is sulfonated with $SO_3$ or chlorosulfonic acid at from 20 to 80°C, the resulting acyloxybenzenesulfonic acid is subsequently acylated with an acyl chloride of the formula Cl—COOR, where R has the meanings stated for formula II, and in order to effect neutralization, the resulting liquid acryloxybenzenesulfonic acid of the formula I is then combined with an aqueous solution of an alkali metal or alkaline earth metal hydroxide, carbonate or bicarbonate in water at from 0 to 60°C, with thorough mixing, so that a pH of from 3.0 to 5.5 is maintained, and, if desired, the salt obtained is isolated from the aqueous solution in a conventional manner.

2. A process as claimed in claim 1, wherein a phenyl ester of the formula II is sulfonated directly with $SO_3$ or chlorosulfonic acid at from 25 to 55°C.

3. A process as claimed in claim 1, wherein the sulfonation of a phenyl ester of the formula II is carried out in the presence of from 1 to 20 mole%, based on $SO_3$ or chlorosulfonic acid, of a complexing agent for $SO_3$ or chlorosulfonic acid, at from 25 to 55°C.

4. A process as claimed in claim 1, wherein the reaction mixture containing the acyloxybenzenesulfonic acid is esterified at from 40 to 50°C with a carboxylic acid chloride of the formula Cl—COR, where R has the meanings given for formula I, in an equimolar amount based on the free phenolic OH groups present.

5. A process as claimed in claim 1, wherein the neutralization is carried out at from 10 to 40°C.

6. A process as claimed in claim 1 and/or 5, wherein the neutralization is carried out in the presence of from 1 to 2% by weight, based on the acyloxybenzenesulfonic acid, of a soluble phosphate, phosphite or tartrate, of a complexing agent for heavy metal salts or of a polymer obtained from acrylic acid and/or maleic acid.

7. A process as claimed in claims 1, 5 or 6, wherein the neutralized solution is oxidatively lightened with from 0.05 to 2% by weight, based on the solids content, of sodium perborate, hydrogen peroxide or sodium peroxodisulfate, at from 40 to 80°C, in the course of from 5 to 15 hours.

8. A process as claimed in claim 1, wherein sulfonation is carried out using a phenyl ester of the formula II prepared by azeotropic esterification of phenol with a carboxylic acid of the formula R—COOH, where R has the meanings given for formula I, in the presence of an aromatic hydrocarbon and of a strongly acidic esterification catalyst.